# EUROPEAN PATENT SPECIFICATION

(11) **EP 3 395 304 B1**
(45) Date of publication and mention of the grant of the patent: **11.02.2026**
(21) Application number: 16878816.4
(22) Date of filing: 21.12.2016
(51) Int. Cl.: A61F 5/02, B25J 11/00, A61F 5/01, A61H 3/00, A63B 21/00

(54) **BODY SUPPORT DEVICE**
KÖRPERUNTERSTÜTZUNGSVORRICHTUNG
DISPOSITIF DE SUPPORT DE CORPS

(30) Priority: 25.12.2015 JP 2015254701
(43) Date of publication of application: 31.10.2018
(73) Proprietor: Nitto Co., Ltd., Yokohama-shi, Kanagawa 236-0002 (JP)
(72) Inventor: FUJISAWA, Hideyuki, Yokohama-shi Kanagawa 236-0002 (JP); NISHIMURA, Hiroaki, Tokyo 104-0033 (JP); KAWAHIRA, Hiroshi, Shimotsuke-shi Tochigi 329-0498 (JP); NAKAMURA, Ryoichi, Chiba-shi Chiba 263-8522 (JP)
(74) Representative: Boult Wade Tennant LLP
(86) International application number: PCT/JP2016/088218
(87) International publication number: WO 2017/110929

(56) References cited:
- WO-A1-2008/113321
- DE-A1- 2 921 227
- DE-A1- 3 213 282
- JP-A- 2005 144 156
- JP-A- 2007 160 029
- JP-A- 2009 011 429
- JP-A- 2009 011 429
- JP-A- 2009 195 291
- JP-A- 2010 268 978
- JP-A- H05 137 742
- JP-A- H11 290 360
- JP-A- S60 501 143
- KR-A- 20100 089 013
- US-A- 5 399 154
- US-A1- 2005 276 657
- US-A1- 2006 100 559
- US-A1- 2007 213 648
- US-A1- 2008 039 756
- US-A1- 2011 112 452
- US-A1- 2012 143 112
- US-B2- 9 101 451

## Description

### Technical Field

The present invention relates to body support devices.

### Background Art

Working in a particular position, such as a semi-crouched position, places burden on the back and knees of the worker, and the reduction of such work burden has been demanded. In response to this demand, devices have been developed which enable users to not only maintain a predetermined position but also to return to a normal position and walk. For example, a wearable chair is known whereby the user can maintain a seated position and then stand up easily and walk (see, for example, Non-patent Document 1).

### Citation List

KR20100089013A discloses an ankle brace attachable to knee brace. A rotatory spring has a bent part at both ends. A knee rotation angle central axis of the knee brace comprises a stopper, a guide groove, a fixing hole, a rising slide groove, a rotary sliding groove and a rubber band. A rising protrusion and a rotatory protrusion are installed at a rigid frame of the knee brace. A fixing protrusion and a fixing ring are protrusively installed to the rigid frame of the lower support of the knee brace. A connection frame has a multi-staged groove in a sawtooth shape. KR20100089013A discloses the preamble of claims 1 and 4.

### Non-patent Document

Non-patent Document 1: "SAKIDORI - You can sit without a chair! This is really a wearable chair!" [online], [date of search: December 25, 2015], internet <URL: http://sakidori.co/article/57332>

### Summary

### Technical Problem

However, the conventional techniques have some drawbacks; for example, wearers cannot always maintain their desired positions. For example, it is difficult for wearers to maintain a position in which the center of gravity of the wearer's body is located ahead of the foot position.

Under these circumstances, an object of the present invention is to provide a body support device that enables a user who wears the device to maintain a desired position, particularly with the user's knees bent.

### Solution to Problem

According to an aspect of the present invention, a body support device in accordance with claim 1 is provided.

Furthermore, according to an aspect of the present invention, a body support device in accordance with claim 4 is provided.

### Advantageous Effects of Invention

According to the present invention, the wearer of the device can maintain a desired position with their knees bent.

### Brief Description of Drawings

Fig. 1 is a perspective illustration schematically showing an example of a body support device.
Fig. 2 is an illustration showing an example of the overall appearance of a body support device.
Fig. 3 is an illustration showing an example of a body support device in use.
Fig. 4 is an illustration showing an example of a body support device in a rotated state.
Fig. 5 is an illustration schematically showing a body support device in a non-rotated state and in a rotated state.
Fig. 6 is an illustration showing an example of a first stop mechanism before its assembly.
Fig. 7 is an illustration showing an example of a first stop mechanism after its assembly.
Fig. 8 is an illustration showing a state in which rotation is restricted by a first stop mechanism.
Fig. 9 is an illustration for explaining the outline of a mechanism that enables the change of position of a stop pin.
Fig. 10 is an illustration showing an example of an adjustment mechanism for adjustment of length.
Fig. 11 is an illustration for explaining another configuration of a body support device.
Fig. 12 is an illustration for explaining another configuration example of a first stop mechanism.
Fig. 13 is an illustration for explaining the configuration of a right/left rotary part.

### Description of Embodiments

Hereinafter, embodiments of the present invention will be described with reference to the attached drawings. It should be noted that the embodiments described below are presented by way of example only, and do not intend to exclude various modifications and technical applications not expressly mentioned in the below description. It should also be noted that, in the below description related to the drawings, the same or similar portions will be given the same or similar reference numerals. Each of the drawings is a schematic illustration and the sizes and dimensional ratios, etc., shown in the drawings do not necessarily correspond to the actual sizes and dimensional ratios, etc. Further, the drawings may include portions in which the dimensional relationships and ratios are shown differently between the drawings.

### [Embodiments]

### <Overall configuration>

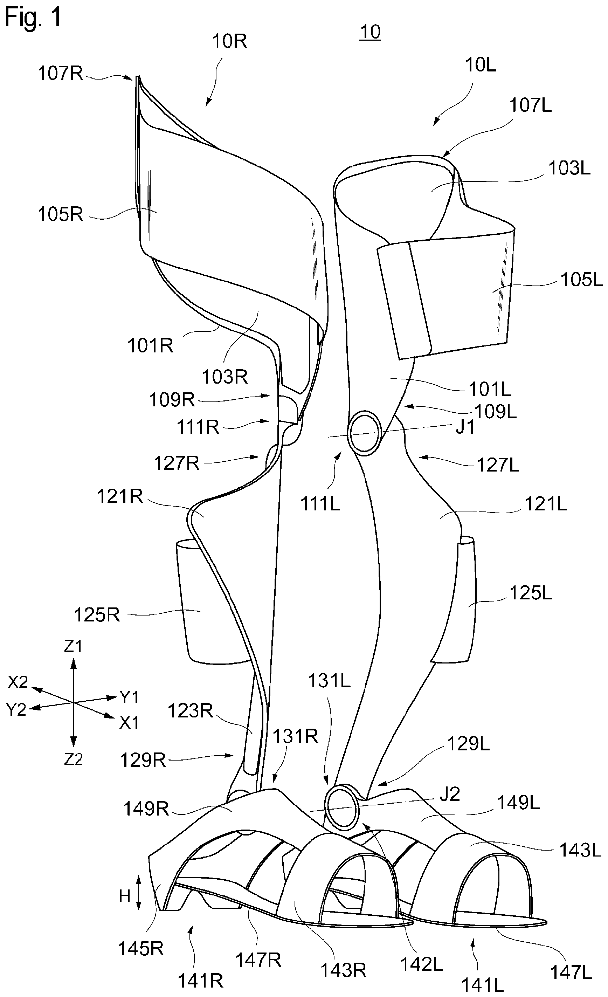

Fig. 1 is a perspective illustration schematically showing an example of a body support device 10 for legs, which is a body support device according to an embodiment of the present invention. The body support device 10 shown in Fig. 1 has a body support device 10R for the right leg and a body support device 10L for the left leg, which are formed independently of one another. As illustrated in Fig. 1, the body support device 10R for the right leg and the body support device 10L for the left leg are basically symmetrical and have similar configurations. In the below description, each element of the configuration will be denoted by a reference number only when it is unnecessary to distinguish between right and left, while each element will be denoted by a reference number with "R" for right and "L" for left if it is necessary to distinguish between right and left.

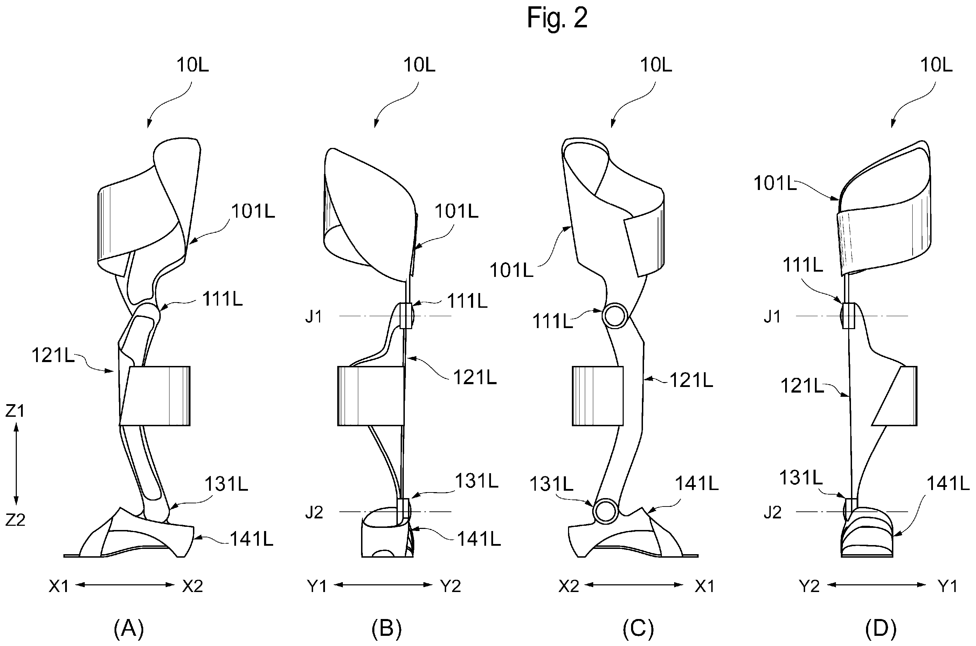

Fig. 2 is an illustration showing an example of the overall appearance of the body support device 10L. Figs. 2(A), 2(B), 2(C) and 2(D) are a right side view, a back view, a left side view, and a front view of the body support device 10L, respectively.

In Figs. 1 and 2, the Z1 direction is vertically upward, the Z2 direction is vertically downward, the X1 direction is a direction forward of the leg, the X2 direction is a direction backward of the leg, the Y1 direction is a direction outward of the leg when it is the left leg, and the Y2 direction is a direction inward of the leg when it is the left leg. The configuration of the body support device 10 will now be described with reference to Figs. 1 and 2.

The body support device 10 has a first support part 101, a second support part 121, a grounding part 141, a first rotary part 111 and a second rotary part 131.

The first support part 101 is attached to the back of the thigh of the wearer. More specifically, the first support part 101 has an end 107 and another end 109, and the first support part 101 is attached to the thigh back of the wearer so that the end 107 is located close to the hip of the wearer and the end 109 is located close to the knee of the wearer. For example, the end 107 may be an upper end portion of the first support part 101 in the vertical direction (i.e., an end portion in the direction from the thigh toward the base of the leg) and the end 109 may be a lower end portion in the same (vertical) direction (i.e., an end portion in the direction from the thigh toward the knee).

The second support part 121 is attached to the shin of the wearer. More specifically, the second support part 121 has an end 127 and another end 129, and the second support part 121 is attached to the shin of the wearer so that the end 127 is located close to the knee of the wearer and the end 129 is located closed to the foot of the wearer. For example, the end 127 may be an upper end portion of the second support part 121 in the vertical direction (i.e., an end portion in the direction from the shin toward the knee) and the end 129 may be a lower end portion in the same (vertical) direction (i.e., an end portion in the direction from the shin toward the malleolus).

The grounding part 141 is located close to the foot of the wearer when the device is worn. For example, the grounding part 141 may have an end 142 and other ends 145 and 147, and the end 142 may be located close to the malleolus of the wearer. Furthermore, in a state in which the rotation of the first rotary part 111 or the rotation of the second rotary part 131 is at least restricted, which will be described later, the ends 145 and 147 of the grounding part 141 come into contact with the ground. For example, the end 142 may represent an upper end portion in the vertical direction and the ends 145 and 147 may represent a lower end portion in the vertical direction.

The first rotary part 111 connects the end 109 of the first support part 101 and the end 127 of the second support part 121. The first rotary part 111 rotates one of the first and second support parts 101 and 121 relative to the other according to an angle of the shin with respect to the back of the thigh of the wearer. Furthermore, the first rotary part 11 restricts the rotation in a direction that brings the thigh back of the wearer closer to the calf to within a predetermined angle.

The second rotary part 131 connects the end 129 of the second support part 121 and the end 142 of the grounding part 141. The second rotary part 131 rotates one of the second support part 121 and the grounding part 141 relative to the other according to an angle of the foot of the wearer with respect to the shin. The second rotary part 131 restricts the rotation in a direction that brings the shin of the wearer closer to the instep of the foot to within a predetermined angle.

With the above-described configuration, the first and second rotary parts 111 and 131 in the body support device 10 restrict the rotation of the first support part 101, the second support part 121 and the grounding part 141 at two points, i.e., around the knee and around the malleolus, to within a predetermined angle, so that the first and second support parts 101 and 121 are locked relative to the grounding part 141. As a result, the weight borne by the thigh and the lower leg of the wearer can be received by the first and second support parts 101 and 121 that support such thigh and lower leg, and the weight can further be received by the grounding part 141 on the ground. Consequently, the wearer can stably maintain a particular position (or a particular posture), such as a semi-crouched position with their knees bent, while reducing the load applied to the muscles in the thigh and the lower leg.

To be more specific, when the wearer takes, for example, a position (a first position) in which the center of gravity of their body is placed ahead of their foot position and when the first and second rotary parts 111 and 131 are in locked positions, the burden borne by the thigh of the wearer is supported by the first support part 101, while the weight of the wearer is supported by the grounding part 141 that is in contact with the ground. In this way, the load applied to the thigh muscles can be reduced. Furthermore, when the wearer takes, for example, the first position, and when the second rotary part 131 is in a locked position, the burden borne by the lower leg of the wearer is supported by the second support part 121, while the weight of the wearer is supported by the grounding part 141 that is in contact with the ground. In this way, the load applied to the muscles in the lower leg can be reduced.

Moreover, since the body support device 10 is attached independently to the right and left legs, the motion range of one leg is not limited by the motion range of the other leg. As a result, the wearer is allowed to move their legs easily, e.g., the wearer can easily move their legs laterally (to open the legs). In addition, when the wearer loses their balance, the balance can easily be restored, so that a fall, etc., of the wearer can be suppressed. Hereinafter, each part of the device will be more specifically described.

The first support part 101 extends in an arc from the inner side of the thigh (the inner side surface of the thigh and the inner side surface of the lower leg will hereinafter be referred to as the "inner side") along the back of the thigh near the hip, and is shaped to cover the back of the thigh. The first support part 101 has such a shape that it extends vertically (i.e., toward the hip) from the end 109, which is close to the lower end on the inner side of the thigh, and then turns to the back of the thigh toward the outer side of the thigh (the outer side surface of the thigh and the outer side surface of the lower leg will hereinafter be referred to as the "outer side").

Furthermore, the upper or lower edge of the first support part 101 extends in an arc from the end 107, which is at the upper end on the outer side of the thigh, toward the end 109, which is at the lower end on the inner side of the thigh. The first support part 101 has an arc-like cross-sectional shape in the XY plane and is formed so as to cover at least one third of the thigh.

The corner of the end 107 of the first support part 101 is rounded. This configuration can prevent the wearer from having pain when the end 107 is attached near their hip. The end 109 of the first support part 101 extends vertically with a predetermined width (about 10-15 cm) so that it can be connected easily with the end 127 of the second support part 121.

The first support part 101 is preferably formed of a thin material with a high strength, such as a carbon material. This ensures that the first support part 101 has strength and flexibility while being light in terms of weight.

The first support part 101 also has a first elastic member (e.g., a slow rebound material) 103 on a surface which is brought into contact with the back of the thigh, and a first assisting part 105 for assisting the attachment to the thigh. The first elastic member may be a stretchable member.

The first elastic member 103 is formed of a material having slow rebound properties, such as urethane, silicon, and polypropylene. Since it is the first elastic member 103 that is in direct contact with the thigh when the wearer takes, for example, a semi-crouched position, the above configuration eliminates stress on the wearer even if their thigh is in contact with the first support part 101 for a long time, and moreover, such configuration can absorb the difference in the body size of each wearer. The first elastic member 103 may be provided on the entire surface of the first support part 101, or be provided in a specific area of the surface of the first support part 101. Alternatively, several elastic members may be distributed over the first support part 101.

The first assisting part 105 has a predetermined width in the vertical direction and is formed in an arc so as to cover the portion extending from the front surface on the inner side of the thigh to the back surface on the outer side of the thigh. The first assisting part 105 is provided detachably from the first support part 101. The first assisting part 105 and the first support part 101 are provided so as to be attached by surrounding the thigh. The first assisting part 105 may be, for example, an elastic or stretchable band-like member made of fabric or the like. The first assisting part 105 does not need to have a high stretching property, and it is sufficient for the first assisting part 105 to have a slightly stretching property. With this configuration, the wearer does not have an uncomfortable feeling in the attachment via the first assisting part 105 and, furthermore, the first support part 101 can be placed not too far from their thigh. Moreover, the first assisting part 105 is detachable so that it can be washed and the color, material, etc., thereof may be changed according to the wearer's preference.

The first support part 101 may have a given form other than the form shown in Figs. 1 and 2, as long as it is designed to cover the thigh from the inner side to the outer side (or from the outer side to the inner side) and the surface of the first support part 101 which is in contact with the thigh extends in an arc so as to prevent the first support part 101 from being displaced relative to the thigh.

The second support part 121 extends in an arc from the inner side of the lower leg along the shin, and is shaped so as to partly or entirely cover the shin. The upper or lower edge of the second support part 121 is formed such that the horizontal length of the arc gradually increases, reaches a maximum and then decreases gradually from the end 127, which is at the upper end on the inner side of the lower leg, toward the end 129, which is at the lower end on the inner side of the lower leg. The second support part 121 has a predetermined thickness sufficient to support the lower leg. The surface of the second support part 121 that is in contact with the shin has an arc-like cross-sectional shape in the XY plane, and the second support part 121 is formed so that the shin is covered by a portion with the longest arc.

The corners of the second support part 121, other than the ends 127 and 129, are rounded. This configuration can prevent the wearer from having pain when such corner portions are attached to their shin. The ends 127 and 129 of the second support part 121 extend vertically with a predetermined width so that the ends can be easily connected with other members. For example, the width of the end 127 (about 10-15 cm) is greater than the width of the end 129 (about 5-10 cm).

The second support part 121 is preferably formed of a thin material with high strength, such as a carbon material, and has similar functions/effects to those of the first support part 101. For example, the second support part 121 has a second elastic member (e.g., a slow rebound material) 123 on a surface which is brought into contact with the shin, and a second assisting part 125 for assisting the attachment to the lower leg.

The second elastic member 123 is formed of a material having slow rebound properties, such as urethane, silicon, and polypropylene. Since it is the second elastic member 123 that is in direct contact with the lower leg when the wearer takes, for example, a semi-crouched position, the above configuration eliminates stress on the wearer even if their lower leg is in contact with the second support part 121 for a long time, and moreover, such configuration can absorb the difference in the body size of each wearer.

The second elastic member 123 may be provided on the entire surface of the second support part 121, or be provided in a specific area of the surface of the second support part 121. Alternatively, several elastic members may be distributed over the second support part 121. The materials of the first and second elastic members 103 and 123 may be the same or different.

The second assisting part 125 has a predetermined width in the vertical direction and is formed in an arc so as to cover the portion extending from the front surface on the inner side of the lower leg to the back surface on the inner side of the lower leg. The second assisting part 125 is provided detachably from the second support part 121. The second assisting part 125 and the second support part 121 are provided so as to be attached by surrounding the lower leg. The second assisting part 125 has similar functions/effects to those of the first assisting part 105. Thus, the wearer does not have an uncomfortable feeling in the attachment via the second assisting part 125 and, furthermore, the second support part 121 can be placed not too far from their lower leg. Moreover, the second assisting part 125 is detachable so that it can be washed and the color, material, etc., thereof may be changed according to the wearer's preference.

The second support part 121 may have a given form other than the form shown in Figs. 1 and 2, as long as it is designed to cover the lower leg from the inner side to the outer side (or from the outer side to the inner side) of the lower leg and the surface of the second support part 121 that is in contact with the lower leg extends in an arc so as to prevent the second support part 121 from being displaced relative to the lower leg.

The grounding part 141 comes into contact with the ground when the wearer bends their knees, and the grounding part 141 bears the load applied thereon via the first and second support parts 101 and 121. The grounding part 141 is attached to, for example, the foot of the wearer and it is preferably shaped to cover the foot entirely or partly from the sole to the instep of the foot. The shape, etc., of the grounding part 141 is, however, not limited to that described above. For example, the grounding part 141 may be shaped so as to come into contact with the ground at least at two points in the longitudinal direction of the foot, instead of coming into contact with the ground via the entire surface thereof. With this configuration, the grounding part 141 can bear the weight of the wearer at two or more points so that a predetermined position can be stably maintained. More preferably, such two or more points include a point in a direction from the malleolus to the heel and a point in a direction from the malleolus to the toe.

The grounding part 141 has an end 142 which is located at the upper end thereof, and has other ends 145 and 147 which are located at the lower end thereof. The end 142 of the grounding part 141 is connected with the second support part 121 via the second rotary part 131. The ends 145 and 147 are portions of the grounding part 141 that come into contact with the ground, and such portions apply load to the ground and receive reaction from the ground.

The grounding part 141 has: a third assisting part 143 for assisting the attachment to the instep of the foot; a bottom part 145 serving as a portion that comes into contact with the ground; a placement part 147 on which the foot is placed; and a body part 149.

The third assisting part 143 has a band-like shape with a predetermined width and is formed to surround the foot from the instep to the sole in such a manner that the third assisting part 143 is arched on the side of a surface of the placement part 147 on which the wearer's foot is placed (i.e., the surface opposite to the surface of the placement part 147 that comes into contact with the ground). The third assisting part 143 may be configured so that the two ends thereof are respectively connected with the ends of the placement part 147 in the width direction of the foot. The third assisting part 143 has similar functions/effects to those of the first assisting part 105 and the second assisting part 125. That is, the third assisting part 143 is stretchable and detachable. It should be noted that the grounding part 141 may be configured so as to have no such third assisting part 143.

The position, width and other dimensions of the third assisting part 143 are shown in Fig. 1 by way of example only. The third assisting part 143 may be provided at a given location in the grounding part 141 and may have a given width and other dimensions, as long as the third assisting part 143 is sufficient to secure the foot of the wearer on the placement part 147. The grounding part 141 is preferably configured such that at least one of a bare foot, a foot with a sock, or a foot with a shoe can be received in the grounding part 141.

The bottom part 145 is formed of an elastic rubber material, such as elastomer. This provides an improved grip against the ground when the wearer tries to maintain a certain position, and furthermore, this makes less sound while walking. The bottom part 145 is preferably light-colored, such as being colored in gray. If the bottom part 145 leaves a stain on the ground, such light color will make it hard to spot the staining.

The placement part 147 has one end on which the heel of the wearer is placed and the other end on which the toe of the wearer is placed. Preferably, the placement part 147 is formed such that, in a state where the placement part 147 is in contact with the ground, the height (or the distance) H (H1) from the ground to the surface where the foot is placed at the one end of the placement part 147 is greater than the height (H2) from the ground to the surface where the foot is placed at the other end. The placement part 147 may also be configured so that the lowest point of the heel of the wearer is located higher than the lowest point of the toe portion by a value equal to or greater than a predetermined value. With this configuration, even when the heel of the wearer is raised above the ground in order to maintain, for example, a semi-crouched position, the load applied to the portion near the toe can be distributed to the entire sole so that the position can be more stably maintained.

For example, the height H from the ground to the surface where the foot is placed at the heel portion of the placement part 147 (i.e., at the one end of the placement part 147) is preferably 30 mm. The height, 30 mm, is just an example, and a different optimum value may be determined by experiment, etc., or a mechanism capable of adjusting the height may be provided. By providing such mechanism for adjusting the height of the heel, the placement part 147 can be adjusted, for example, to be almost parallel to the ground while walking; whereas the placement part 147 can be adjusted to raise the heel relative to the ground while maintaining, for example, a semi-crouched position.

The bottom part 145 is formed at one end of a surface of the placement part 147 that contacts or faces the ground. Alternatively, the bottom part 145 may be formed on such surface in its entirety, or a plurality of bottom parts 145 may be distributed over such surface. With the aid of the thickness of the bottom part 145, the height H1 at the one end of the placement part 147 may be arranged so as to be higher than the height H2 at the other end of the placement part 147.

The body part 149 has a band-like portion extending from one end of the placement part 147 to the other so as to cover a portion of the instep near the ankle, and another band-like portion extending from a portion close to the center of the first band-like portion toward the toe of the placement part 147. The body part 149 may be formed of, for example, a carbon material. In the example shown in Fig. 1, the third assisting part 143 is provided at an end portion of the body part 149 that is close to the toe, and the second rotary part 131 is connected to the band-like portion that covers the ankle. It should be noted that the form of the body part 149 shown in Fig. 1 is just an example, and a given form can be adopted as long as it is easy to wear on the foot.

It should be noted that the grounding part 141 may have a given configuration, as long as the grounding part 141, in its overall configuration, is easy to wear on the foot, able to substantially secure the foot with the third assisting part 143 and the body part 149, and not difficult to detach from the foot.

The first, second and third assisting parts 105, 125 and 143 may be formed of, for example, a material with good elasticity and durability, such as chloroprene rubber, and it is preferable to use a hook-and-loop fastener (Magic Tape^{®}), which is often used in, for example, guards for athletes.

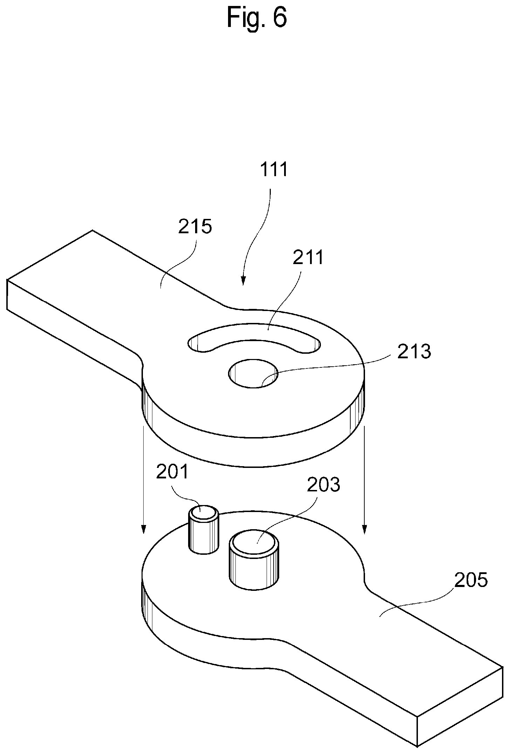

For example, the first rotary part 111 may connect the first support part 101 and the second support part 121 rotatably about a reference line J1 of a first axis of rotation shown in Fig. 1. The first rotary part 111 has a first stop mechanism so as to restrict the rotation to within a predetermined angle. The details of the first stop mechanism will be described later, with reference to Figs. 6-9.

For example, the second rotary part 131 may connect the second support part 121 and the grounding part 141 rotatably about a reference line J2 of a second axis of rotation shown in Fig. 1. The second rotary part 131 has a second stop mechanism so as to restrict the rotation to within a predetermined angle. The structure of the second stop mechanism is basically the same as that of the first stop mechanism. It is preferable for the first and second stop mechanisms to be made of a material with high strength, such as a metallic material, so that they do not deform even if they receive the weight of a human body.

It is also preferable that the first rotary part 111 rotatably connect the first support part 101 and the second support part 121 at a position around the knee of the wearer, and that the second rotary part 131 rotatably connect the second support part 121 and the grounding part 141 at a position around the malleolus (or the ankle) of the wearer. With this configuration, the angle of the device around the knee and the angle of the device around the ankle can be fixed, so that the wearer can stably maintain a desired position with their knees bent.

The first and second stop mechanisms may be provided with an angular adjustment mechanism with which the angle for restriction can be selected from among a plurality of angles. Furthermore, each of the angles given by the first stop mechanism may be associated with a corresponding one of the angles given by the second stop mechanism. In such a case, the first and second stop mechanisms may have an identification mechanism for identifying the correlation between an angle of the first stop mechanism and an angle of the second stop mechanism. For example, such identification mechanism may be configured so that angle A of the knee and associated angle A of the ankle can be identified upon angular adjustment by adding the same mark or using the same color in the respective adjustment mechanisms. With this configuration, combinations of the angles of the knee and the angles of the ankle can be presented to the wearer in an easy-to-understand manner. When the body support device 10 is worn, the body balance of the wearer has a relationship with the angle of the knee and the angle of the ankle, and accordingly, the above-mentioned combinations may be combinations to stabilize the body balance of the wearer.

In the case where the first stop mechanism of the first rotary part 111 has a locked state in which the rotation is restricted to within a predetermined angle and an unlocked state in which such restriction of rotation is released, the first stop mechanism may have an identification mechanism for identifying the locked and unlocked states. For example, such identification mechanism may be configured so as to identify the locked and unlocked states by color. With this configuration, the wearer or persons around the wearer can immediately understand that the body support device 10 is locked or unlocked and the wearer can be prevented from attempting to maintain a certain position with the device unlocked, leading to improvements in terms of safety.

The first rotary part 111 and the second rotary part 131 are preferably arranged at the inner side of the leg, as shown in Fig. 1, although they may be arranged at the front, the outer side, or the back of the leg. With such arrangement, the wearer can easily see the rotary parts and adjust them at certain angles, as compared to the case in which the rotary parts are at the outer side or the back of the leg. Moreover, the rotary parts located at the side of the leg are not likely to be an obstacle during walking, etc., as compared to rotary parts located at the back or front of the leg. In addition, the rotary parts located at the inner side of the leg will result in the wearer manipulating the inner portion of their leg in order to adjust the angles, and this means that the wearer can make angular adjustments with natural movements without bearing a load. Moreover, the muscles of the thigh usually grow outward, and thus, by placing the rotary parts at the inner side of the leg, the applicability of the device can be broadened so that a wide variety of people can wear and use the device.

If the first rotary part 111 and the second rotary part 131 also rotate in a direction opposite to the direction of rotation under the above-indicated angular restriction, the rotary parts may have no angular restriction for such rotation in the opposite direction. With this configuration, each of the first rotary part 111 and the second rotary part 131 rotate freely in the opposite direction so that the body support device 10 can be folded into a compact size and is convenient to carry around.

The first support part 101 and the second support part 121 may be connected spirally via the first rotary part 111. This configuration can make the body support device 10 difficult to detach from the leg of the wearer when the wearer moves their leg back and forth and right to left. Likewise, the grounding part 141 may be connected with the second support part 121 spirally via the second rotary part 131. This configuration can make it more difficult to detach the body support device 10 from the leg.

Furthermore, it is preferable that the surface of the carbon material used for the first support part 101, the second support part 121 and the grounding part 141 be, for example, white in color. With this configuration, when the body support device 10 is used, for example, during surgery, blood and other stains attached to the device become easy to notice, so that the device can be handled while paying attention to hygiene.

As illustrated in Figs. 1 and 2, the body support device 10L, as a whole, is formed by connecting the respective parts at the inner side of the leg along the Z1-Z2 direction so that a spiral shape is formed based on the inner side of the leg. In addition to the above-mentioned effect to suppress detachment of the body support device 10L, this configuration provides the body support device 10L with an aesthetic design.

Furthermore, as shown in Fig. 2, the first support part 101L and the second support part 121L are rotatable about the reference line J1 of the first axis of rotation. Taking the body support device 10L shown in Fig. 2(C) as an example, the first support part 101L rotates freely in the X1 direction based on the first rotary part 111L, while the first support part 101L rotates in the X2 direction with a predetermined angular restriction.

As shown in Fig. 2, the second support part 121L and the grounding part 141 are rotatable about the reference line of the second axis of rotation J2. Taking the body support device 10L shown in Fig. 2(C) as an example, the second support part 121L rotates freely in the X2 direction based on the second rotary part 131L, while the second support part 121L rotates in the X1 direction with a predetermined angular restriction.

### <Usage>

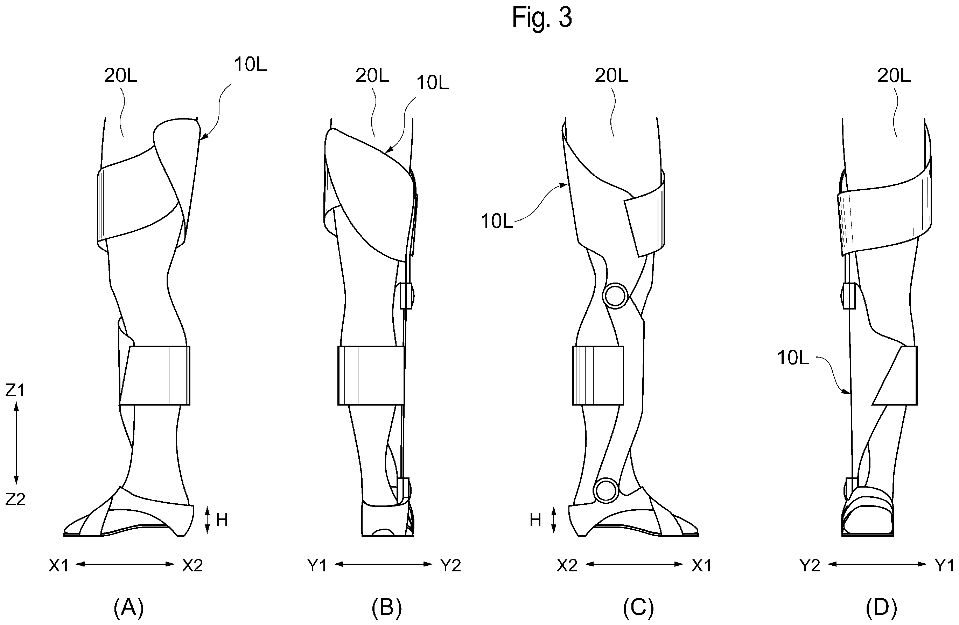

Fig. 3 is an illustration showing an example of the body support device 10L in use. Reference numeral 20L in Fig. 3 denotes the left leg of the wearer. In the example shown in Fig. 3, the leg 20L is a bare leg (foot), but trousers and/or shoes may be worn on the legs (feet).

As illustrated in Fig. 3, the heel of the left leg is at a height H away from the ground, while the toe portion of the left leg is in contact with the ground. With this height H, when the wearer takes a predetermined position, such as a semi-crouched position, and moves with the heels above the ground, the load applied to a portion near the toe can be distributed to the entire sole and the position can be more stably maintained.

Furthermore, as illustrated in Fig. 3, a band portion of the first assisting part 105L, together with the first support part 101L, enables secure attachment to the thigh of the left leg 20L of the wearer. Such band portion of the first assisting part 105L also enables size adjustment.

As illustrated in Fig. 3, a band portion of the second assisting part 125L, together with the second support part 121L, enables secure attachment to the lower leg of the left leg 20L of the wearer. Such band portion of the second assisting part 125L also enables size adjustment.

As illustrated in Fig. 3, a band portion of the third assisting part 143L, together with the placement part 147, enables secure attachment to the instep of the left foot of the wearer. Such band portion of the third assisting part 143L also enables size adjustment.

As shown in Fig. 3, the body support device 10L looks stylish when it is worn on the wearer, and such impression can encourage the use of the body support device 10L.

### <Rotation Example>

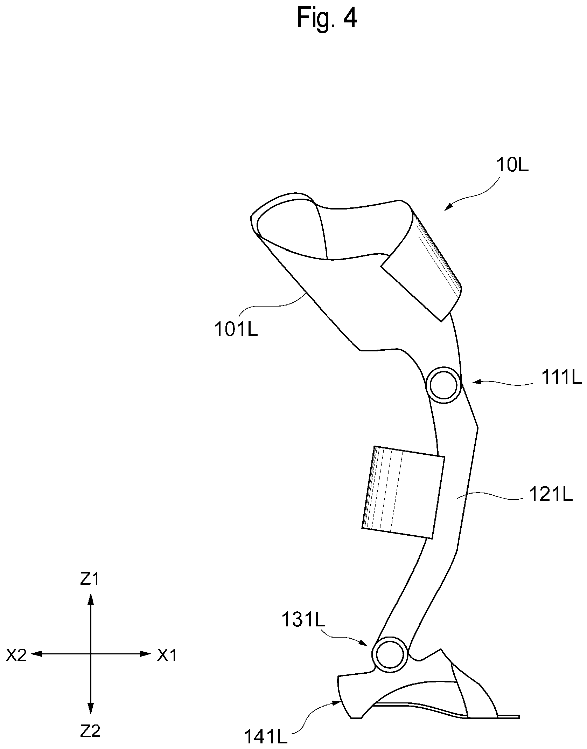

Next, the manner in which the body support device 10L rotates will be described. Fig. 4 is an illustration showing an example of the body support device 10L in a rotated state. In the example shown in Fig. 4, the first support part 101L and the second support part 121L rotate in a direction that brings the thigh back and the calf closer to each other, and the second support part 121L and the grounding part 141L rotate in a direction that brings the shin and the instep closer to each other.

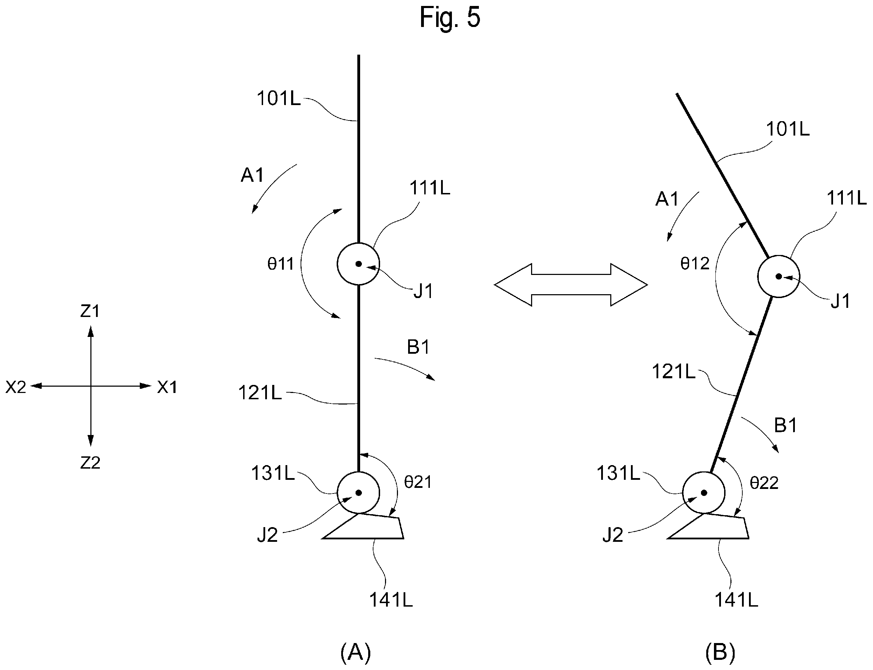

Fig. 5 schematically illustrates the non-rotated and rotated states of the body support device 10. Fig. 5(A) shows the non-rotated state, in which, in the direction A1 that brings the thigh back and the calf closer to each other, the angle θ11 formed by the first support part 101L and the second support part 121L is about 180 degrees approximately around the first axis of rotation J1. Further, in the direction B1 that brings the shin and the instep closer to each other, the angle θ21 formed by the second support part 121L and the grounding part 141L is about 120 degrees approximately around the axis of rotation J2.

Fig. 5(B) shows the rotated state, in which, in the direction A1 that brings the thigh back and the calf closer to each other, the angle θ12 formed by the first support part 101L and the second support part 121L is about 150 degrees. Further, in the direction B1 that brings the shin and the instep closer to each other, the angle θ22 formed by the second support part 121L and the grounding part 141L is about 90 degrees. In the example shown in Fig. 5(B), the angles θ12 and θ22 indicate restricted angles. In the example shown in Fig. 5(B), the wearer can maintain a first position (a position in which the center of gravity of the wearer's body is located ahead of the foot position). It should be noted here that a plurality of angles may be set for each of the angles θ12 and θ22, so as to stop rotation in a stepwise manner. It should also be noted that the above-mentioned angles are presented by way of example only.

### <Stop Mechanism>

Next, the first stop mechanism will be described. Since the second stop mechanism has almost the same mechanism as that of the first stop mechanism, description for the second stop mechanism will be omitted. The below description will briefly explain the stop mechanism. It should also be noted that such stop mechanism may be implemented by using a known stop mechanism, as long as it is able to restrict rotation to within a particular angle.

Fig. 6 is an illustration showing an example of the first stop mechanism before its assembly. In the example shown in Fig. 6, the first rotary part 111 is comprised of members 205 and 215. The members 205 and 215 each have a circular shape at one end portion thereof, and the first stop mechanism is provided in such circular portions.

For example, another end portion of the member 205, which is rectangular in shape, may be connected with the second support part 121 via screws, welding, etc., and another end portion of the member 215, which is also rectangular in shape, may be connected with the first support part 101 via screws, welding, etc. The member 205 may be formed integrally with the second support part 121 as an end portion thereof, and the member 215 may be formed integrally with the first support part 101 as an end portion thereof. The first stop mechanism may include, for example, a stop pin 201 and a rotation restricting groove 211.

The members 205 and 215 may be exchanged in terms of their positions. Specifically, a stop pin 201 and a rotation shaft 203 may be provided in the member 215, and a rotation restricting groove 211 and a rotation hole 213 may be provided in the member 205.

The member 205 has, at the circular end portion thereof, a rotation shaft 203 arranged at the center of the circular end portion and shaped like a pin, and a stop pin 201 arranged at the peripheral portion. The member 215 has, at the circular end portion thereof, a circular rotation hole 213 arranged at the center of the circular end portion, and an arc-shaped rotation restricting groove 211 arranged at the peripheral portion. The rotation shaft 203 may serve as, for example, the first axis of rotation.

When the respective circular end portions of the members 205 and 215 are assembled together, the rotation shaft 203 is inserted into the rotation hole 213 and the stop pin 201 is inserted into the rotation restricting groove 211.

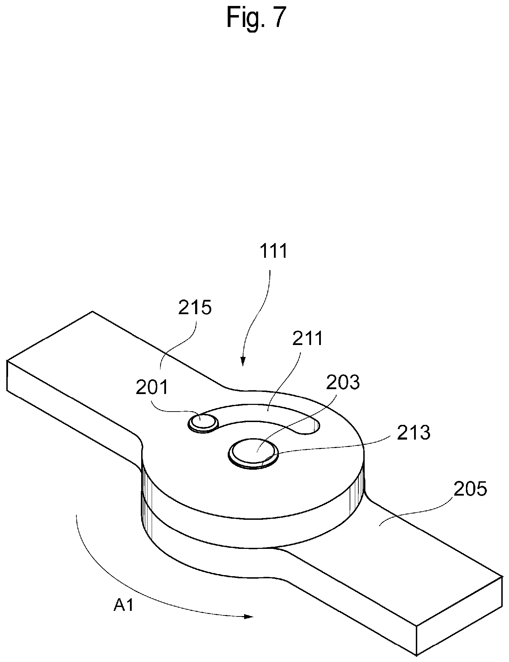

Fig. 7 is an illustration showing an example of the first stop mechanism after its assembly. In the example shown in Fig. 7, the rotation shaft 203 is inserted into the rotation hole 213 and the stop pin 201 is inserted into the rotation restricting groove 211 in the first rotary part 111. In this example of Fig. 7, the direction A1 indicates a direction in which the thigh back and the calf are approaching each other.

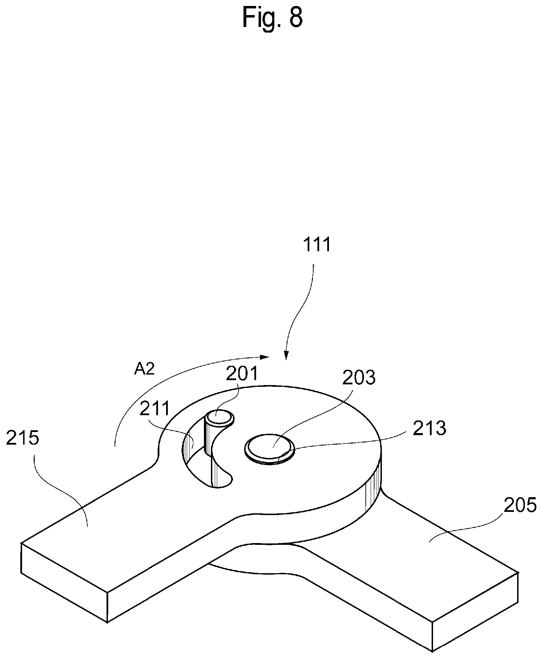

Fig. 8 is an illustration showing a state in which rotation is restricted by the first stop mechanism. In the example shown in Fig. 8, the member 215 rotates in the direction A1, and such rotation is then restricted when the stop pin 201 is located at an edge of the rotation restricting groove 211. The direction A2 may be, for example, a direction in which the thigh back and the calf are becoming distant from each other.

The range of the rotation is determined according to the length of the arc of the rotation restricting groove 211. For example, a shorter arc of the rotation restricting groove 211 narrows the rotation range, and a longer arc of the rotation restricting groove 211 increases the rotation range.

The position of the stop pin 201 may be configured so as to be changeable with a gear, etc. (not shown in the drawings), in order to enable the rotation to be restricted to within given angles. In this configuration, a state in which the position of the stop pin 201 is changeable is called an unlocked state, and a state in which the position of the stop pin 201 is fixed is called a locked state. Such unlocked and locked states are preferably distinguishable by, for example, color.

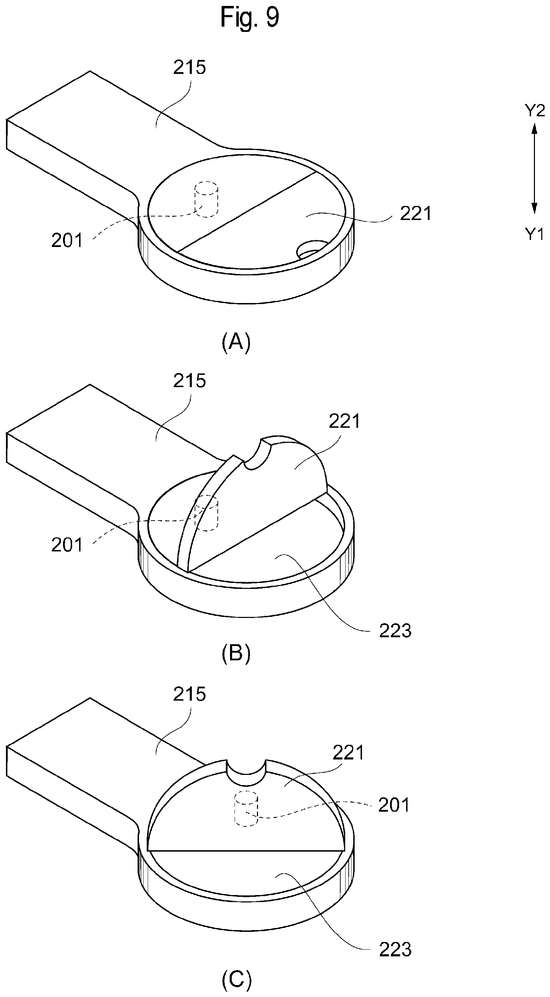

Fig. 9 is an illustration for explaining an outline of the mechanism that enables the change in the position of the stop pin 201. The example shown in Fig. 9 describes a simplified theory. In the example shown in Fig. 9, the stop pin 201 is provided on a surface of the member 215 that faces in the Y1 direction, and the stop pin 201 extends in the Y1 direction. In the example shown in Fig. 9, the member 215 has a knob part 221 on a surface that faces in the Y2 direction. It should be noted that the rotation shaft 203 is not shown in the example of Fig. 9.

In the example shown in Fig. 9(A), the knob part 221 is laid down and the knob part 221 in this state does not rotate (or turn). That is, Fig. 9(A) represents a locked state. In this locked state, the position of the stop pin 201 will not be changed.

In the example shown in Fig. 9(B), the knob part 221 is raised upright and the knob part 221 in this state is able to turn clockwise and counter clockwise about the center thereof. That is, Fig. 9(B) represents an unlocked state. In this unlocked state, the position of the stop pin 201 will be changed as the knob part 221 turns. Here, a surface 223, which is visible when the knob part 221 is raised upright, has a particular color, such as red. This arrangement can inform the wearer and persons around the wearer of the fact that the device is currently in an unlocked state.

In the example in Fig. 9(C), the knob part 221 has turned about 45 degrees clockwise from the state shown in Fig. 9(B). As shown in Fig. 9(C), the stop pin 201 has also turned about 45 degrees in the clockwise direction along with the turn of the knob part 221, so that the position of the stop pin 201 has been changed. By changing the position of the stop pin 201, the wearer can stop rotation at a desired angle selected from among a plurality of angles.

Furthermore, the wearer may be informed of the specific restriction angle selected by applying multiple colors on the surface 223 and using such colors. The mechanism shown in Fig. 9 may also be provided in the second stop mechanism. In such a case, colors, marks, etc., formed on the surface 223 may be used in order to make the correlation between one of the angles of restriction given by the first stop mechanism and one of the angles of restriction given by the second stop mechanism understandable, if such correlation exists.

### <Adjustment Mechanism>

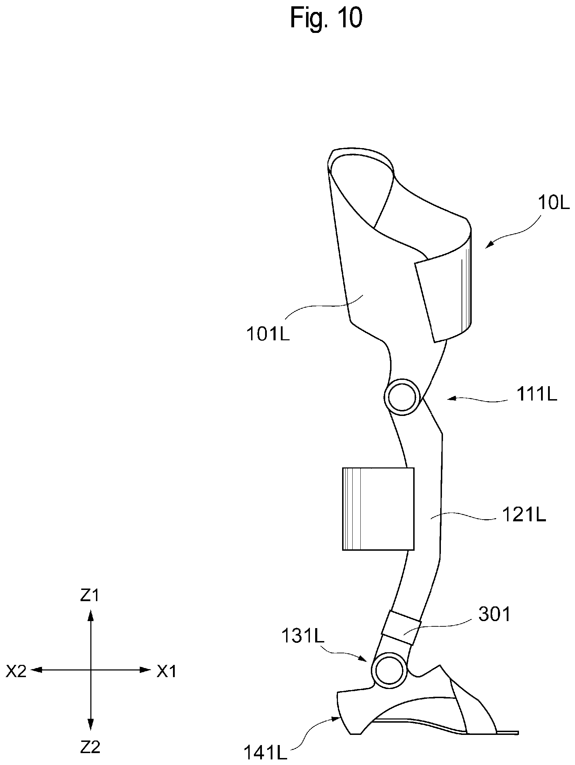

Fig. 10 is an illustration showing an example of the adjustment mechanism for making length adjustment. In the example shown in Fig. 10, an adjustment mechanism 301 is provided between the second support part 121 and the second rotary part 131. The adjustment mechanism 301 adjusts the vertical length of the body support device 10. Since it is desirable for the first rotary part 111 to be located near the knee, the position of the first support part 101 can preferably be adjusted according to the position of the wearer's knee. Furthermore, in order to make the body support device 10 applicable to wearers whose legs are different in length, it is preferable that the length of the device be adjustable at the lower leg portion thereof. In light of this, the example shown in Fig. 10 is designed such that the adjustment mechanism 301 is provided at either the end 129 of the second support part 121 or the end 142 of the grounding part 141 so as to adjust the position at which the one end is connected to the other end. With this arrangement, the wearer can set an appropriate length of the body support device 10 according to the length of the wearer's own leg (e.g., the length of the wearer's lower leg), leading to improved user-friendliness of the body support device 10.

### <Modifications>

The body support device 10 may be configured such that the lower end of the second support part 121 in the vertical direction comes into contact with the ground, without providing the second rotary part 131 and the grounding part 141. In this configuration, the second support part 121 needs to have strength sufficient to endure the load.

The above-mentioned drawings describe an example in which the grounding part 141 is attached to the foot of the wearer, but the grounding part 141 may be modified so as not be attached to the foot. For example, the grounding part 141 may be configured so as to be rotatable at the side of the foot without being in contact with the ground when the wearer is standing or walking, and when the wearer bends their knees to take, for example, a semi-crouched position, the grounding part 141 may come into contact with the ground and the rotation thereof may be restricted.

Alternatively, the grounding part 141 may be provided on the front surface of the second support part 121 (i.e., the surface opposite to the surface that is attached to the shin), so that the grounding part 141 is contained in the second support part 121 when the wearer is standing or walking; whereas the grounding part 141 hangs down vertically from the second support part 121 to bring the lower end thereof into contact with the ground when the wearer bends their knees to take a semi-crouched position.

Although the first rotary part 111 and/or the second rotary part 131 is provided on one side (e.g., the inner side) of the leg in the above-described example, an additional rotary part may be provided on the opposing side (e.g., the outer side) so as to improve strength.

The upper end of the first support part 101 may extend further to the hip portion, without stopping near the thigh back. Furthermore, the first support part 101 may be formed of a first supporting rod member associated with the thighbone and a first receptor member for receiving the thigh back. The second support part 121 may also be formed of a second supporting rod member associated with the bone of the lower leg and a second receptor member for receiving the lower leg.

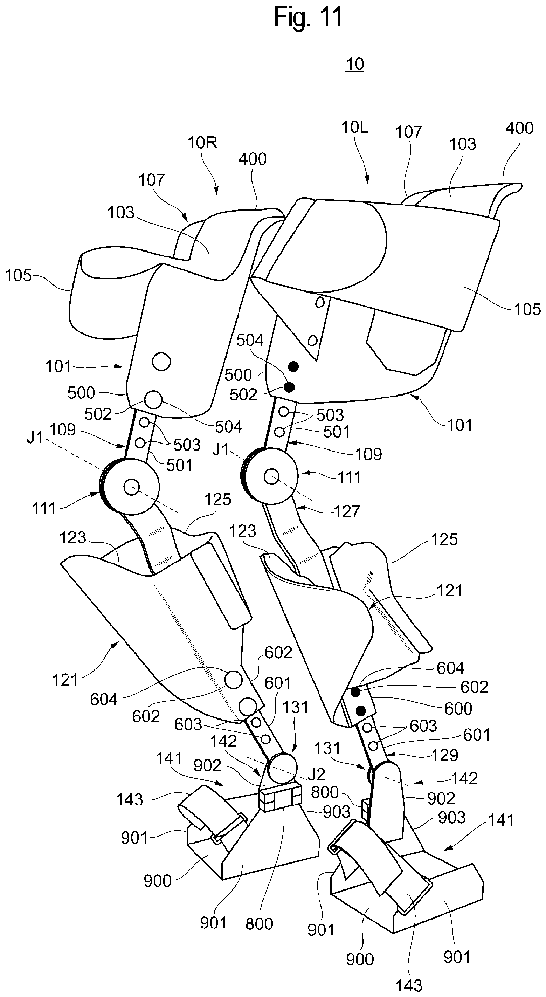

Although both the rotation of the first support part 101 and the rotation of the second support part 121 are restricted in the above-described embodiment, the rotation of the first support part 101 alone may be restricted without restricting the rotation of the second support part 121. In such a case, for example, as illustrated in Fig. 11, a body support device 10 is configured so as to include: a first support part 101 which is attached to the back of the thigh of the wearer in such a manner that an end 107 thereof is located close to the hip of the wearer and another end 109 thereof is located close to the knee of the wearer; a second support part 121 which is attached to the shin of the wearer in such a manner that an end 127 thereof is located close to the knee of the wearer and another end 129 thereof is located close to the foot of the wearer; a grounding part 141 that is located close to the foot of the wearer; a first rotary part 111 which connects the end 109 of the first support part 101 with the end 127 of the second support part 121 and which rotates either the first support part 101 or the second support part 121 relative to the other, according to an angle of the shin with respect to the thigh back of the wearer; and a second rotary part 131 which connects the end 129 of the second support part 121 with an upper end 142 of the grounding part 141 and which rotates either the second support part 121 or the grounding part 141 relative to the other, according to an angle of the foot with respect to the shin of the wearer. The first rotary part 111 is able to restrict the rotation in a direction that brings the thigh back and the calf of the wearer closer to each other to within a predetermined angle.

In this example, since the body support device 10 restricts the rotation of the first rotary part 111 at a desired angle, the first support part 101 and the second support part 121 are locked relative to one another when the wearer bends their knees and a load is applied to the first support part 101. As a result, the weight of the wearer can be borne by the first support part 101, the second support part 121 and the first rotary part 111. Further, the weight of the wearer that has been applied to the second support part 121 can be received by the second support part 121, the grounding part 141 and the second rotary part 131. Since the rotation of the second rotary part 131 is not restricted, the wearer can maintain a position with their knees bent while keeping balance back and forth with the second rotary part 131 at the center of the balance. In other words, the wearer can stably maintain a predetermined position (or posture), such as a semi-crouched position with their knees bent, while reducing load applied to the muscles in the thigh, the lower leg, etc.

In the body support device 10 according to the above-described example, the first support part 101 may have an extension part 400 which is formed by smoothly curving the end 107 of the first support part 101 backward and extending the curved end horizontally backward. With this configuration, the hip portion of the wearer can be received by the extension part 400, and accordingly, the wearer can take a more comfortable seated position. In addition, the stability of the seated position can also be improved.

For example, the first support part 101 and the second support part 121 may each be provided with an adjustment feature for changing the length. For example, the first support part 101 may have an upper, first member 500 and a lower, second member 501. The first member 500 and the second member 501 may be provided with holes 502 and holes 503, respectively, the holes being arranged vertically in the first and second members. The length of the first support part 101 can be adjusted by aligning a desired one of the holes 502 of the first member 500 with a desired one of the holes 503 of the second member 501, inserting a screw 504 into the aligned holes 502 and 503 and then fastening the screw 504. Likewise, the second support part 121 may have an upper, first member 600 and a lower, second member 601. The first member 600 and the second member 601 may be provided with holes 602 and holes 603, respectively, the holes being arranged vertically in the first and second members. The length of the second support part 121 can be adjusted by aligning a desired one of the holes 602 of the first member 600 with a desired one of the holes 603 of the second member 601, inserting a screw 604 into the aligned holes 602 and 603 and then fastening the screw 604.

The first rotary part 111 has a first stop mechanism to restrict rotation, as described above (see Figs. 6-9). The first stop mechanism may be configured so that the restricted angle of rotation can be selected from a plurality of angles, as described above. Furthermore, the first rotary part 111 may be provided with another first stop mechanism.

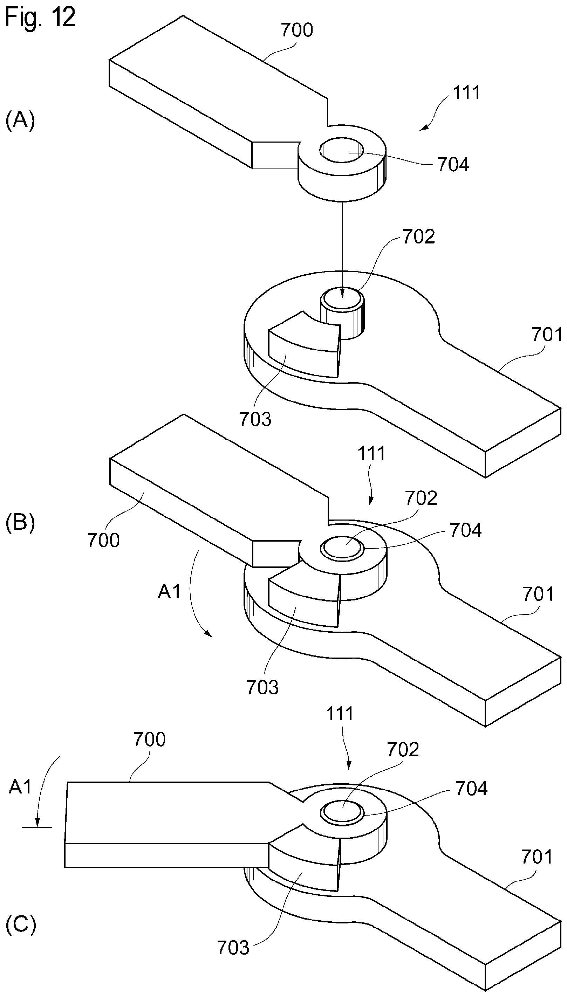

Fig. 12(A) is an illustration showing an example of another first stop mechanism before its assembly. In the example shown in Fig. 12(A), the first rotary part 111 is comprised of a member 700 connected with the first support part 101 and a member 701 connected with the second support part 121. An end of the member 701 is circular in shape, and a pin-shaped rotation shaft 702 is formed at the center of the circular portion. Further, an arc-shaped stop block 703 is provided on the circular portion of the member 701. The member 700 has a rotation hole 704 formed at an end of the member 700. The rotation shaft 702 of the member 701 is fitted into the rotation hole 704 of the member 700. With this arrangement, the member 700 can rotate with respect to the member 701 about the rotation shaft 702, as illustrated in Fig. 12(B). Furthermore, the rotation of the member 700 in the direction A1 (the direction in which the thigh back and the calf are approaching each other) is restricted by the stop block 703 as shown in Fig. 12(C). The moving range may be selected as appropriate by changing the internal angle of the arc of the stop block 703 or changing the position where the stop block 703 is provided.

The second rotary part 131 may have the same configuration as that of the first rotary part 111 shown in Figs. 12(A) to 12(C), in which, however, no stop block 703 is formed. With this configuration, the rotation of the second rotary part 131 will not be restricted.

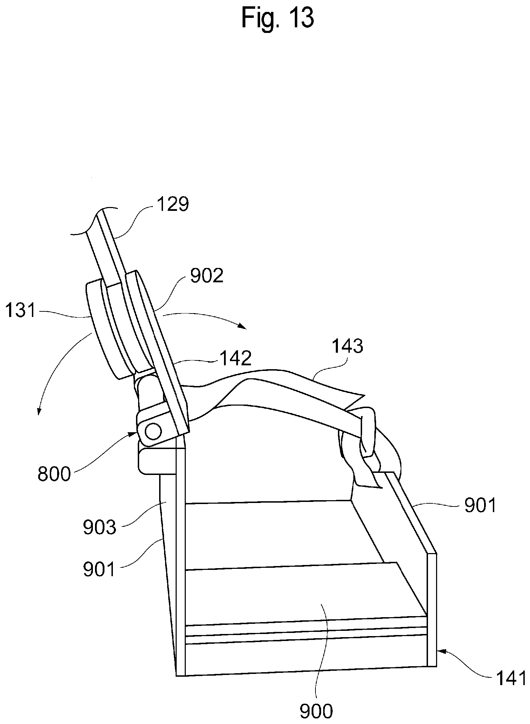

The body support device 10 illustrated in Fig. 11 has a right/left rotary part 800 for rotating the grounding part 141 in the right/left direction relative to the second support part 121. The grounding part 141 has a placement part 900 on which the foot of the wearer is placed, and a side part 901. For example, the side part 901 has a first, upper member 902 serving as the end 142 of the grounding part 141, and a second, lower member 903 which is formed so as to extend upward from the placement part 900. The right/left rotary part 800 may be, for example, a hinge mechanism which is provided between the first member 902 and the second member 903 and connects the first and second members 902 and 903 so as to allow rotation in the right/left direction, as shown in Fig. 13. With this configuration, the placement part 900, which is a part of the grounding part 141, can be rotated in the right/left direction with respect to the second support part 121. As a result, for example, when the wearer spreads their feet to the sides, the placement part 900 of the grounding part 141 can be placed parallel to the ground and a considerable amount of contact area with the ground can be ensured, so that the wearer can stabilize their position.

In the above-described configuration of the body support device 10 that does not restrict the rotation of the second support part 121, elements other than those particularly mentioned above are similar to those in the configuration of the body support device 10 that involves restriction of the rotation of the second support part 121, and therefore, the description of such elements will be omitted here. Furthermore, every element which is applicable to such example of the body support device 10 that does not restrict the rotation of the second support part 121 may be applied to the example of the body support device 10 that restricts the rotation of the second support part 121.

### Reference Signs List

- 10: Body support device
- 101: First support part
- 111: First rotary part
- 121: Second support part
- 131: Second rotary part
- 141: Grounding part

## Claims

1. A body support device (10) for supporting a body of a wearer such that the wearer can maintain a desired position with their knees bent, comprising:
a first support part (101) having a first end (107) and a second end (109) and for attachment to a thigh back of the wearer so that the first end (107) is located close to a hip of the wearer and the second end (109) is located close to a knee of the wearer;
a second support part (121) having a first end (127) and a second end (129) and for attachment to a shin of the wearer so that the first end (127) is located close to the knee of the wearer and the second end (129) is located close to a foot of the wearer;
a grounding part (141) for location close to the foot of the wearer;
a first rotary part (111) that connects the second end (109) of the first support part (101) with the first end (127) of the second support part (121) and for rotating one of the first and second support parts (101, 121) relative to the other according to an angle of the shin with respect to the thigh back of the wearer; and
a second rotary part (131) that connects the second end (129) of the second support part (121) with an upper end (142) of the grounding part (141) and for rotating one of the second support part (121) and the grounding part (141) relative to the other according to an angle of the foot with respect to the shin of the wearer, wherein:
the first rotary part (111) restricts the rotation in a direction for bringing the thigh back and a calf of the wearer closer to each other to within a predetermined angle;
the second rotary part (131) restricts the rotation in a direction for bringing the shin and an instep of the foot of the wearer closer to each other to within a predetermined angle;
the grounding part (141) is for coming into contact with a ground in a state in which the rotation of the first rotary part (111) or the rotation of the second rotary part (131) is restricted; and **characterised in that**
the first and second rotary parts (111, 131) restrict the rotation such that the first and second support parts (101, 121) are locked relative to the grounding part (141) for the body support device (10) to support the weight of the wearer for the wearer to maintain the desired position with their knees bent.

2. The body support device (10) according to claim 1, wherein:
the first rotary part (111) has a first stop mechanism whereby a restricted angle of the rotation can be selected from among a plurality of angles; and
the second rotary part (131) has a second stop mechanism whereby a restricted angle of the rotation can be selected from among a plurality of angles.

3. The body support device (10) according to claim 2, wherein the angles selectable by the first stop mechanism have a correlation with angles selectable by the second stop mechanism, and the first and second stop mechanisms have an identification mechanism for identifying the correlation.

4. A body support device (10) for supporting a body of a wearer such that the wearer can maintain a desired position with their knees bent, comprising:
a first support part (101) having a first end (107) and a second end (109) and for attachment to a thigh back of the wearer so that the first end (107) is located close to a hip of the wearer and the second end (109) is located close to a knee of the wearer;
a second support part (121) having a first end (127) and a second end (129) and for attachment to a shin of the wearer so that the first end (127) is located close to the knee of the wearer and the second end (129) is located close to a foot of the wearer;
a grounding part (141) for location close to the foot of the wearer;
a first rotary part (111) that connects the second end (109) of the first support part (101) with the first end (127) of the second support part (121) and for rotating one of the first and second support parts (101, 121) relative to the other according to an angle of the shin with respect to the thigh back of the wearer; and
a second rotary part (131) that connects the second end (129) of the second support part (121) with an upper end (142) of the grounding part (141) and for rotating one of the second support part (121) and the grounding part (141) relative to the other according to an angle of the foot with respect to the shin of the wearer,
wherein the first rotary part (111) restricts the rotation in a direction for bringing the thigh back and a calf of the wearer closer to each other to within a predetermined angle, **characterised in**
**that** the first support part (101) and the second support part (121) are locked relative to one another for the body support device (10) to support the weight of the wearer for the wearer to maintain the desired position with their knees bent.

5. The body support device (10) according to claim 4, wherein the first rotary part (111) has a first stop mechanism whereby a restricted angle of the rotation can be selected from among a plurality of angles.

6. The body support device (10) according to any one of claims 1 to 5, wherein the first rotary part (111) has an identification mechanism for indicating a locked state in which a state of restricting the rotation to within the predetermined angle is maintained.

7. The body support device (10) according to any one of claims 1 to 6, wherein the second end (109) of the first support part (111) and the first end (127) of the second support part (121) are connected spirally via the first rotary part (111).

8. The body support device (10) according to any one of claims 1 to 7, further comprising an adjustment mechanism (301) provided at either the second end (129) of the second support part (121) or the upper end (142) of the grounding part (141) for adjusting a position at which the second end (129) and the upper end (142) are connected with each other.

9. The body support device (10) according to any one of claims 1 to 8, wherein:
the first support part (101) has a first elastic member (103) on a surface for coming into contact with the thigh back; and
the second support part (121) has a second elastic member (123) on a surface for coming into contact with the shin.

10. The body support device (10) according to any one of claims 1 to 9, wherein the first and second rotary parts (111, 131) are for disposal on an inner side of a leg.

11. The body support device (10) according to any one of claims 1 to 10, wherein:
the grounding part (141) has a placement part (147) for placement of the foot of the wearer thereon;
the placement part (147) has a first end, for being in a direction toward a heel of the wearer, and a second end, for being in a direction toward a toe of the wearer; and
the placement part (147) is configured such that, when the placement part (147) is in contact with the ground, the first end of the placement part (147) has a longer distance from the ground than a distance of the second end of the placement part (147) from the ground.

12. The body support device (10) according to any one of claims 1 to 11, wherein:
the first support part (101) has a first assisting part (105) for attaching the first support part (101) to surround a thigh portion;
the second support part (121) has a second assisting part (125) for attaching the second support part (121) to surround a lower leg portion;
the first assisting part (105) is detachable from the first support part (101), or the second assisting part (125) is detachable from the second support part (121).

13. The body support device (10) according to any one of claims 1 to 12, further comprising a right/left rotary part (800) that rotates the grounding part (141) in a right/left direction relative to the second support part (800).

## Patentansprüche

1. Körper-Unterstützungsvorrichtung (10) zum Unterstützen eines Körpers eines Trägers, so dass der Träger eine gewünschte Position mit angewinkelten Knien beibehalten kann, umfassend:
einen ersten Unterstützungsteil (101), welcher ein erstes Ende (107) und ein zweites Ende (109) aufweist und zum Anbringen an einem hinteren Schenkel des Trägers vorgesehen ist, so dass das erste Ende (107) nahe einer Hüfte des Trägers angeordnet ist und das zweite Ende (109) nahe einem Knie des Trägers angeordnet ist;
einen zweiten Unterstützungsteil (121), welcher ein erstes Ende (127) und ein zweites Ende (129) aufweist und zum Anbringen an einem Schienbein des Trägers vorgesehen ist, so dass das erste Ende (127) nahe dem Knie des Trägers angeordnet ist und das zweite Ende (129) nahe einem Fuß des Trägers angeordnet ist;
einen Bodenkontakt-Teil (141) für ein Anordnen nahe dem Fuß des Trägers;
einen ersten Rotationsteil (111), welcher das zweite Ende (109) des ersten Unterstützungsteils (101) mit dem ersten Ende (127) des zweiten Unterstützungsteils (121) verbindet, und zum Rotieren von einem aus den ersten und zweiten Unterstützungsteilen (101, 121) relativ zu dem anderen gemäß einem Winkel des Schienbeins bezüglich des hinteren Schenkels des Trägers; und
einen zweiten Rotationsteil (131), welcher das zweite Ende (129) des zweiten Unterstützungsteils (121) mit einem oberen Ende (142) des Bodenkontakt-Teils (141) verbindet, und zum Rotieren von einem aus dem zweiten Unterstützungsteil (121) und dem Bodenkontakt-Teil (141) relativ zu dem anderen gemäß einem Winkel des Fußes bezüglich des Schienbeins des Trägers, wobei:
der erste Rotationsteil (111) die Rotation in einer Richtung zum Bringen des hinteren Schenkels und einer Wade des Trägers näher zueinander auf innerhalb eines vorbestimmten Winkels beschränkt;
der zweite Rotationsteil (131) die Rotation in einer Richtung zum Bringen des Schienbeins und eines Rists des Fußes des Trägers näher zueinander auf innerhalb eines vorbestimmten Winkels beschränkt;
der Bodenkontakt-Teil (141) zum In-Kontakt-Kommen mit einem Boden in einem Zustand vorgesehen ist, in welchem die Rotation des ersten Rotationsteils (111) oder die Rotation des zweiten Rotationsteils (131) beschränkt ist; und **dadurch gekennzeichnet, dass**
die ersten und zweiten Rotationsteile (111, 131) die Rotation derart beschränken, dass die ersten und zweiten Unterstützungsteile (101, 121) relativ zu dem Bodenkontakt-Teil (141) verriegelt sind, damit die Körper-Unterstützungsvorrichtung (10) das Gewicht des Trägers für den Träger unterstützt, um die gewünschte Position mit angewinkelten Knien beizubehalten.

2. Körper-Unterstützungsvorrichtung (10) nach Anspruch 1, wobei:
der erste Rotationsteil (111) einen ersten Stopp-Mechanismus aufweist, wodurch ein beschränkter Winkel der Rotation aus einer Mehrzahl von Winkeln ausgewählt werden kann; und
der zweite Rotationsteil (131) einen zweiten Stopp-Mechanismus aufweist, wodurch ein beschränkter Winkel der Rotation aus einer Mehrzahl von Winkeln ausgewählt werden kann.

3. Körper-Unterstützungsvorrichtung (10) nach Anspruch 2, wobei die Winkel, welche durch den ersten Stopp-Mechanismus auswählbar sind, eine Korrelation mit Winkeln aufweisen, welche durch den zweiten Stopp-Mechanismus auswählbar sind, und die ersten und zweiten Stopp-Mechanismen einen Identifikationsmechanismus zum Identifizieren der Korrelation aufweisen.

4. Körper-Unterstützungsvorrichtung (10) zum Unterstützen eines Körpers eines Trägers, so dass der Träger eine gewünschte Position mit angewinkelten Knien beibehalten kann, umfassend:
einen ersten Unterstützungsteil (101), welcher ein erstes Ende (107) und ein zweites Ende (109) aufweist und zum Anbringen an einem hinteren Schenkel des Trägers vorgesehen ist, so dass das erste Ende (107) nahe einer Hüfte des Trägers angeordnet ist und das zweite Ende (109) nahe einem Knie des Trägers angeordnet ist;
einen zweiten Unterstützungsteil (121), welcher ein erstes Ende (127) und ein zweites Ende (129) aufweist und zum Anbringen an einem Schienbein des Trägers vorgesehen ist, so dass das erste Ende (127) nahe dem Knie des Trägers angeordnet ist und das zweite Ende (129) nahe einem Fuß des Trägers angeordnet ist;
einen Bodenkontakt-Teil (141) für ein Anordnen nahe dem Fuß des Trägers;
einen ersten Rotationsteil (111), welcher das zweite Ende (109) des ersten Unterstützungsteils (101) mit dem ersten Ende (127) des zweiten Unterstützungsteils (121) verbindet, und zum Rotieren von einem aus den ersten und zweiten Unterstützungsteilen (101, 121) relativ zu dem anderen gemäß einem Winkel des Schienbeins bezüglich des hinteren Schenkels des Trägers; und
einen zweiten Rotationsteil (131), welcher das zweite Ende (129) des zweiten Unterstützungsteils (121) mit einem oberen Ende (142) des Bodenkontakt-Teils (141) verbindet, und zum Rotieren von einem aus dem zweiten Unterstützungsteil (121) und dem Bodenkontakt-Teil (141) relativ zu dem anderen gemäß einem Winkel des Fußes bezüglich des Schienbeins des Trägers, wobei
der erste Rotationsteil (111) die Rotation in einer Richtung zum Bringen des hinteren Schenkels und einer Wade des Trägers näher zueinander auf innerhalb eines vorbestimmten Winkels beschränkt; **dadurch gekennzeichnet, dass** der erste Unterstützungsteil (101) und der zweite Unterstützungsteil (121) relativ zueinander verriegelt sind, damit die Körper-Unterstützungsvorrichtung (10) das Gewicht des Trägers für den Träger unterstützt, um die gewünschte Position mit angewinkelten Knien beizubehalten.

5. Körper-Unterstützungsvorrichtung (10) nach Anspruch 4, wobei der erste Rotationsteil (111) einen ersten Stopp-Mechanismus aufweist, wodurch ein beschränkter Winkel der Rotation aus einer Mehrzahl von Winkeln ausgewählt werden kann.

6. Körper-Unterstützungsvorrichtung (10) nach einem der Ansprüche 1 bis 5, wobei der erste Rotationsteil (111) einen Identifikationsmechanismus zum Anzeigen eines verriegelten Zustands aufweist, in welchem ein Zustand einer Beschränkung der Rotation auf innerhalb des vorbestimmten Winkels beibehalten wird.

7. Körper-Unterstützungsvorrichtung (10) nach einem der Ansprüche 1 bis 6, wobei das zweite Ende (109) des ersten Unterstützungsteils (111) und das erste Ende (127) des zweiten Unterstützungsteils (121) spiralförmig mittels des ersten Rotationsteils (111) verbunden sind.

8. Körper-Unterstützungsvorrichtung (10) nach einem der Ansprüche 1 bis 7, ferner umfassend einen Einstellmechanismus (301), welcher an entweder dem zweiten Ende (129) des zweiten Unterstützungsteils (121) oder dem oberen Ende (142) des Bodenkontakt-Teils (141) zum Einstellen einer Position bereitgestellt ist, an welcher das zweite Ende (129) und das obere Ende (142) miteinander verbunden sind.

9. Körper-Unterstützungsvorrichtung (10) nach einem der Ansprüche 1 bis 8, wobei:
der erste Unterstützungsteil (101) ein elastisches Element (103) an einer Fläche zum In-Kontakt-Kommen mit dem hinteren Schenkel aufweist; und
der zweite Unterstützungsteil (121) ein zweites elastisches Element (123) an einer Fläche zum In-Kontakt-Kommen mit dem Schienbein aufweist.

10. Körper-Unterstützungsvorrichtung (10) nach einem der Ansprüche 1 bis 9, wobei die ersten und zweiten Rotationsteile (111, 131) für ein Anordnen an einer inneren Seite eines Beins vorgesehen sind.

11. Körper-Unterstützungsvorrichtung (10) nach einem der Ansprüche 1 bis 10, wobei:
der Bodenkontakt-Teil (141) einen Platzierungsteil (147) zum Platzieren des Fußes des Trägers daran aufweist;
der Platzierungsteil (147) ein erstes Ende aufweist, um in einer Richtung zu einer Ferse des Trägers hin zu liegen, sowie ein zweites Ende, um in einer Richtung zu einem Zeh des Trägers hin zu liegen; und
der Platzierungsteil (147) derart eingerichtet ist, dass wenn der Platzierungsteil (147) in Kontakt mit dem Boden ist, das erste Ende des Platzierungsteils (147) eine längere Distanz von dem Boden aufweist als eine Distanz des zweiten Endes des Platzierungsteils (147) von dem Boden.

12. Körper-Unterstützungsvorrichtung (10) nach einem der Ansprüche 1 bis 11, wobei:
der erste Unterstützungsteil (101) einen ersten Assistenzteil (105) zum Anbringen des ersten Unterstützungsteils (101) zum Umgeben eines Schenkelabschnitts aufweist;
der zweite Unterstützungsteil (121) einen zweiten Assistenzteil (125) zum Anbringen des zweiten Unterstützungsteils (121) zum Umgeben eines unteren Beinabschnitts aufweist;
der erste Assistenzteil (105) von dem ersten Unterstützungsteil (101) lösbar ist oder der zweite Assistenzteil (125) von dem zweiten Unterstützungsteil (121) lösbar ist.

13. Körper-Unterstützungsvorrichtung (10) nach einem der Ansprüche 1 bis 12, ferner umfassend einen rechten/linken Rotationsteil (800), welcher den Bodenkontakt-Teil (141) in einer rechten/linken Richtung relativ zu dem zweiten Unterstützungsteil (800) rotiert.

## Revendications

1. Dispositif de soutien corporel (10) permettant de soutenir un corps d'un porteur de sorte que le porteur peut maintenir une position souhaitée avec ses genoux fléchis, comprenant :
une première partie de soutien (101) ayant une première extrémité (107) et une seconde extrémité (109) et destinée à être fixée à un arrière de cuisse du porteur de sorte que la première extrémité (107) est située près d'une hanche du porteur et la seconde extrémité (109) est située près d'un genou du porteur ;
une seconde partie de soutien (121) ayant une première extrémité (127) et une seconde extrémité (129) et destinée à être fixée à un tibia du porteur de sorte que la première extrémité (127) est située près du genou du porteur et la seconde extrémité (129) est située près d'un pied du porteur ;
une partie de positionnement au sol (141) destinée à être située près du pied du porteur ;
une première partie rotative (111) qui relie la seconde extrémité (109) de la première partie de soutien (101) à la première extrémité (127) de la seconde partie de soutien (121) et destinée à faire tourner l'une des première et seconde parties de soutien (101, 121) par rapport à l'autre selon un angle du tibia par rapport à l'arrière de cuisse du porteur ; et
une seconde partie rotative (131) qui relie la seconde extrémité (129) de la seconde partie de soutien (121) à une extrémité supérieure (142) de la partie de positionnement au sol (141) et destinée à faire tourner l'une de la seconde partie de soutien (121) et de la partie de positionnement au sol (141) rapport à l'autre selon un angle du pied par rapport au tibia du porteur, dans lequel :
la première partie rotative (111) limite la rotation dans une direction pour rapprocher l'arrière de cuisse et un mollet du porteur l'un de l'autre dans un angle prédéterminé ;
la seconde partie rotative (131) limite la rotation dans une direction pour rapprocher le tibia et un empeigne du pied du porteur l'un de l'autre dans un angle prédéterminé ;
la partie de positionnement au sol (141) est destinée à entrer en contact avec un sol dans un état dans lequel la rotation de la première partie rotative (111) ou la rotation de la seconde partie rotative (131) est limitée ; et **caractérisé en ce que**
les première et seconde parties rotatives (111, 131) limitent la rotation de sorte que les première et seconde parties de soutien (101, 121) sont verrouillées par rapport à la partie de positionnement au sol (141) afin que le dispositif de soutien corporel (10) soutienne le poids du porteur pour que le porteur maintienne la position souhaitée avec ses genoux fléchis.

2. Dispositif de soutien corporel (10) selon la revendication 1, dans lequel :
la première partie rotative (111) a un premier mécanisme d'arrêt moyennant quoi un angle restreint de la rotation peut être sélectionné parmi une pluralité d'angles ; et
la seconde partie rotative (131) a un second mécanisme d'arrêt moyennant quoi un angle restreint de la rotation peut être sélectionné parmi une pluralité d'angles.

3. Dispositif de soutien corporel (10) selon la revendication 2, dans lequel les angles sélectionnables par le premier mécanisme d'arrêt ont une corrélation avec des angles sélectionnables par le second mécanisme d'arrêt, et les premier et second mécanismes d'arrêt ont un mécanisme d'identification pour identifier la corrélation.

4. Dispositif de soutien corporel (10) pour soutenir un corps d'un porteur de sorte que le porteur peut maintenir une position souhaitée avec ses genoux fléchis, comprenant :
une première partie de soutien (101) ayant une première extrémité (107) et une seconde extrémité (109) et destinée à être fixée à un arrière de cuisse du porteur de sorte que la première extrémité (107) est située près d'une hanche du porteur et la seconde extrémité (109) est située près d'un genou du porteur ;
une seconde partie de soutien (121) ayant une première extrémité (127) et une seconde extrémité (129) et destinée à être fixée à un tibia du porteur de sorte que la première extrémité (127) est située près du genou du porteur et la seconde extrémité (129) est située près d'un pied du porteur ;
une partie de positionnement au sol (141) destinée à être située près du pied du porteur ;
une première partie rotative (111) qui relie la seconde extrémité (109) de la première partie de soutien (101) à la première extrémité (127) de la seconde partie de soutien (121) et destinée à faire tourner l'une des première et seconde parties de soutien (101, 121) par rapport à l'autre selon un angle du tibia par rapport à l'arrière de cuisse du porteur ; et
une seconde partie rotative (131) qui relie la seconde extrémité (129) de la seconde partie de soutien (121) à une extrémité supérieure (142) de la partie de positionnement au sol (141) et destinée à faire tourner l'une de la seconde partie de soutien (121) et de la partie de positionnement au sol (141) rapport à l'autre selon un angle du pied par rapport au tibia du porteur,
dans lequel la première partie rotative (111) limite la rotation dans une direction pour rapprocher l'arrière de cuisse et un mollet du porteur l'un de l'autre dans un angle prédéterminé, **caractérisé en ce que** la première partie de soutien (101) et la seconde partie de soutien (121) sont verrouillées l'une par rapport à l'autre pour que le dispositif de soutien corporelle (10) soutienne le poids du porteur afin que le porteur maintienne la position souhaitée avec ses genoux fléchis.

5. Dispositif de soutien corporel (10) selon la revendication 4, dans lequel la première partie rotative (111) a un premier mécanisme d'arrêt moyennant quoi un angle restreint de la rotation peut être sélectionné parmi une pluralité d'angles.

6. Dispositif de soutien corporel (10) selon l'une quelconque des revendications 1 à 5, dans lequel la première partie rotative (111) a un mécanisme d'identification pour indiquer un état verrouillé dans lequel un état de limitation de la rotation dans l'angle prédéterminé est maintenu.

7. Dispositif de soutien corporel (10) selon l'une quelconque des revendications 1 à 6, dans lequel la seconde extrémité (109) de la première partie de soutien (111) et la première extrémité (127) de la seconde partie de soutien (121) sont connectées en spirale via la première partie rotative (111).

8. Dispositif de soutien corporel (10) selon l'une quelconque des revendications 1 à 7, comprenant en outre un mécanisme de réglage (301) agencé au niveau de la seconde extrémité (129) de la seconde partie de soutien (121) ou au niveau de l'extrémité supérieure (142) de la partie de positionnement au sol (141) pour régler une position à laquelle la seconde extrémité (129) et l'extrémité supérieure (142) sont reliées l'une à l'autre.

9. Dispositif de soutien corporel (10) selon l'une quelconque des revendications 1 à 8, dans lequel :
la première partie de soutien (101) a un premier élément élastique (103) sur une surface destiné à entrer en contact avec l'arrière de cuisse ; et
la seconde partie de soutien (121) a un second élément élastique (123) sur une surface destiné à entrer en contact avec le tibia.

10. Dispositif de soutien corporel (10) selon l'une quelconque des revendications 1 à 9, dans lequel les première et seconde parties rotatives (111, 131) sont destinées à être disposées sur un côté interne d'une jambe.

11. Dispositif de soutien corporel (10) selon l'une quelconque des revendications 1 à 10, dans lequel :
la partie de positionnement au sol (141) a une partie de placement (147) pour placer dessus le pied du porteur ;
la partie de placement (147) a une première extrémité, destinée à être dans une direction vers un talon du porteur, et une seconde extrémité, destinée à être dans une direction vers un orteil du porteur ; et
la partie de placement (147) est configurée de sorte que, lorsque la partie de placement (147) est en contact avec le sol, la première extrémité de la partie de placement (147) présente une distance plus longue par rapport au sol qu'une distance de la seconde extrémité de la partie de placement (147) par rapport au sol.

12. Dispositif de soutien corporel (10) selon l'une quelconque des revendications 1 à 11, dans lequel :
la première partie de soutien (101) a une première partie d'assistance (105) pour attacher la première partie de soutien (101) pour entourer une portion de cuisse ;
la seconde partie de soutien (121) a une seconde partie d'assistance (125) pour attacher la seconde partie de soutien (121) afin d'entourer une portion inférieure de jambe ;
la première partie d'assistance (105) est détachable de la première partie de soutien (101), ou la seconde partie d'assistance (125) est détachable de la seconde partie de soutien (121).

13. Dispositif de soutien corporel (10) selon l'une quelconque des revendications 1 à 12, comprenant en outre une partie rotative droite/gauche (800) qui fait tourner la partie de positionnement au sol (141) dans une direction droite/gauche par rapport à la seconde partie de soutien (800).
